# EUROPEAN PATENT SPECIFICATION

(11) **EP 3 082 913 B1**
(45) Date of publication and mention of the grant of the patent: **04.07.2018**
(21) Application number: 14824453.6
(22) Date of filing: 18.12.2014
(51) Int. Cl.: A61M 5/20, A61M 5/315, A61M 5/48, A61M 5/31

(54) **ASSEMBLY FOR A DRUG DELIVERY DEVICE AND DRUG DELIVERY DEVICE**
ANORDNUNG FÜR EINE ARZNEIMITTELABGABEVORRICHTUNG UND ARZNEIMITTELABGABEVORRICHTUNG
ASSEMBLAGE POUR DISPOSITIF D'ADMINISTRATION DE MÉDICAMENTS ET DISPOSITIF D'ADMINISTRATION DE MÉDICAMENTS

(30) Priority: 20.12.2013 EP 13198707
(43) Date of publication of application: 26.10.2016
(73) Proprietor: Sanofi-Aventis Deutschland GmbH, 65929 Frankfurt am Main (DE)
(72) Inventor: JUGL, Michael, 65926 Frankfurt am Main (DE); TEUCHER, Axel, 65926 Frankfurt am Main (DE); HOLTWICK, Marc, 65926 Frankfurt am Main (DE)
(74) Representative: Weilnau, Carsten
(86) International application number: PCT/EP2014/078417
(87) International publication number: WO 2015/091764

(56) References cited:
- WO-A2-2008/053243
- WO-A2-2009/098502
- WO-A2-2011/043605
- US-A1- 2011 077 595

## Description

The present disclosure relates to an assembly for a drug delivery device. The present disclosure further relates to a drug delivery device. In particular, the disclosure relates to pen-type drug delivery devices.

Pen-type drug delivery devices are used for injections by persons without formal medical training. This is increasingly common for self-treatment among patients having diabetes or the like. By means of a drive mechanism, a bung in a cartridge is displaced such that a drug accommodated in the cartridge is dispensed through a needle.

Prior to injection, the required dose of the drug is set by means of a dose setting mechanism. Common designs of dose setting mechanisms comprise a number of tubular or sleeve-like elements such as a dose setting sleeve, a dose indicating sleeve, a drive sleeve and/or a ratchet sleeve. Such sleeves are often accommodated within and connected to each other. Some devices comprise a power assist, in particular an energy storing member, wherein energy may be stored in the energy storing member during the setting of a dose. This energy may be released during dose delivery.

A power assisted drug delivery device is described in document US 2008/0306446 A1, for example.

WO 2008/053243 A2 discloses a medicine delivery apparatus comprising a trigger. The apparatus is designed so that the user may exert an extra force of variable strength on the trigger during ejection of the medicine, in order to control the speed with which a drive rod is urged forward by the unwinding of a loaded spring.

WO 2009/098502 A2 discloses an injection device comprising a firing button designed to act as a brake on the periphery of a drive gear so as to give the user control over the speed with which the autoinjection cycle is executed. Braking may be effected by friction between the button and the periphery of the drive gear.

US 2011/0077595 A1 discloses a brake mechanism for injection devices. The maximum possible angular velocity of a dispensing movement can be reduced or limited.

Power assisted drug delivery devices are beneficial, in particular when the injection of larger volumes and/or highly viscous fluids is intended. The energy storing member must have a force profile sufficient to enable delivery of the entire dose. This has the impact that at least small doses are dispensed with high force and, therefore, in a fast manner. Fast injections may lead to user discomfort, as the injected tissue is stressed and the user may even feel pain.

It is an object of the present disclosure to provide a drug delivery device having improved properties, e.g. increased user comfort.

This object may be achieved by the subject matter of the independent claims. Advantageous embodiments and refinements are subject matter of the dependent claims.

One aspect relates to an assembly for a drug delivery device. The assembly may comprise a drive mechanism of the device. A drive mechanism may be a mechanism adapted for setting and/or dispensing a dose of drug from the device. The assembly may comprise at least one drive member. The drive member may be a sleeve-like member. The assembly may comprise two, three or more drive members. The drive member may be adapted and arranged to be moved in a delivery direction during a dose delivery operation of the assembly. The drive member may be axially and rotationally moveable during dose delivery. The drive member may be adapted and arranged to be moved in a direction opposite to the delivery direction during a dose setting operation of the assembly. The drive member may be axially and rotationally moveable during dose setting.

The assembly may further comprise an energy storing member, e.g. a spring member. The energy storing member may be a torsion spring, for example. The energy storing member may be adapted and arranged to store energy. The energy may be stored during a dose setting operation. The energy storing member may be adapted and arranged to cause movement of the drive member in the delivery dircetion. In particular, the drive member may be moved by the energy storing member, in particular by means of energy stored in the energy storing member, for delivering a dose of the drug. Energy may be released from the energy storing member when an activation member, e.g. a dose button, is operated by a user. The released energy may cause the drive member to be moved in the delivery direction.

The assembly may further comprise an interaction system. The interaction sytem may be adapted and arranged to generate a friction onto the drive member. In particular, friction may be generated between the interaction system and the drive member. For this purpose, the interaction system and the drive member may be relatively moveable. An amount of friction may regulate a speed of movement of the drive member when drive member is moved in the delivery direction. The greater the friction, the slower the speed of movement of the drive member during a dose delivery operation.

By means of the interaction system, a user can influence the speed of movement of the drive member. In particular, the interaction systems functions as a break of the device. The speed can be adjusted to the individual needs of the user. For example, the speed can be determined by which drug is to be delivered from the device. In this way, provision of a drug delivery device with high user comfort is facilitated.

According to the invention, the interaction system comprises a first interaction member. The interaction system also comprises a second interaction member. The interaction members are moveable with respect to one another. The interaction members are moveable with respect to the drive member. In particular, the first interaction member may be moveable with respect to the second interaction member. Furthermore, the first interaction member may be moveable with respect to the drive member. The second interaction member may be moveable with respect to the first interaction member. Moreover, the drive member may be moveable with respect to the second interaction member.

According to one not-claimed embodiment, the second interaction member is configured to mechanically cooperate with the drive member. The second interaction member may be in direct mechanical contact with the drive member. The second interaction member may abut, preferably permanently abut, the drive member. Upon relative movement between the second interaction member and the drive member, friction may be generated between these two components.

According to the invention, the second interaction member is configured to exert a radial force onto the drive member such that the speed of movement of the drive member is reduced when the drive member is moved in the delivery direction during a dose delivery operation. In particular, the speed of movement of the drive member during dose delivery may be smaller than the speed of movement of the drive member during dose setting. During dose setting, a force exerted by the second interaction member onto the drive member may, thus, be smaller than during dose delivery. Accordingly, the interaction system may not influence a dose setting operation of the assembly. However, the speed of movement during a dose delivery operation may be controllable by means of the interaction system. In this way, an effective and user-friendly device is provided.

According to one embodiment, the first interaction member is adapted to be moved with respect to the second interaction member and with respect to the drive member in a direction which is perpendicular to the delivery direction. In particular, the first interaction member may be adapted to be moved in a radial direction. The amount of friction generated may depend on a radial position of the first interaction member. By means of adjusting the radial position of the interaction member, the speed of movement of the drive member may be effectively controlled.

According to the invention, the second interaction member is adapted to be moved with respect to the first interaction member in an axial direction. The second interaction member is axially moveable between a first position and a second position. The first position may be arranged more proximal, i.e. more away from a dispensing end of the device, than the second position. The second interaction member may be adapted and arranged to be moved towards the first position during a dose setting operation of the assembly. The second interaction member may be adapted and arranged to be moved towards the second position during a dose delivery operation of the assembly. Axial movement of the second interaction member may occur automatically, e.g. due to frictional forces. The second interaction member may be automatically moved between the first and the second position due to mechanical cooperation with the drive member. In this way, the user does not have to influence the axial position of the second interaction member. Thus, the interaction system is very easy to handle. In this way, provision of a safe and user-friendly drug delivery device is facilitated.

According to the invention, the interaction system is adapted and arranged such that the amount of friction increases when the second interaction member is moved towards the second position. Accordingly, the closer the second interaction member is arranged to the second position, the slower the speed of the drive member during dose delivery.

According to one embodiment, the interaction system comprises at least one stop member. The stop member may be adapted and arranged to limit the axial movement of the second interaction member between the first and the second position. The stop member may comprise a flange, or a protrusion, for example. By means of the stop member, axial movement of the second interaction member can be effectively limited.

According to one embodiment, when the second interaction member is arranged in the second position, the first and second interaction members are in mechanical contact with one another. In particular, the interaction members may abut. In the second position, the first interaction member may exert a force, in particular a radially inwardly directed force, onto the second interaction member. The second interaction member may be pushed radially inwardly by the first interaction member. The second interaction member may be pushed towards the drive member. Hence, in the second position, the amount of friction between the second interaction member and the drive member may be increased as compared to the amount of friction when the second interaction member is out of the second position. Thus, when the second interaction member is arranged in the second position, the speed of the drive member may be reduced.

According to one embodiment, when the second interaction member is arranged in the first position, the first and second interaction member are free from mechanical contact with one another. In particular, the interaction members may no longer abut. In the first position, no force may be exerted by the first interaction member onto the second interaction member. As no force is exerted onto the second interaction member, the second interaction member may exert no force or a minor force onto the drive member. Hence, in the first position, the amount of friction between the second interaction member and the drive member is decreased as compared to the amount of friction when the second interaction member is in the second position. Preferably, the amount of friction is decreased to zero when the second interaction member is arranged in the first position. Accordingly, in the first position, the interaction system may not influence the operation of the assembly. In other words, a dose setting operation of the assembly may not be affected by the interaction system.

When the second interaction member is moved towards the second position during dose delivery, the radial force exerted onto the drive member by means of the second interaction member may increase to due mechanical cooperation between the first and second interaction member. In the first position, there may be no mechanical cooperation between the interaction members and, thus, there may be no friction generated between the second interaction member and the drive member. Accordingly, the radial force which is exerted on the drive member when the second interaction member is in the first position may be smaller than the radial force onto the drive member when the second interaction member is moved towards the second position. The amount of the radial force exerted onto the drive member and, thus, the amount of friction, may, thus, depend on the axial position of the second interaction member. The amount of the radial force exerted onto the drive member may further depend on a radial position of the first interaction member. By means of adjusting the positions of the interaction members, the force onto the drive member and, thus, the speed of the drive member may be effectively controlled.

According to an embodiment, the assembly further comprises a piston rod. The piston rod is adapted and arranged to be displaceable in the delivery direction for dispensing a dose of a drug from the device.

The term "drug", as used herein, preferably means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;

or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Movement of the drive member in the delivery direction may be converted into movement of the piston rod in the delivery direction. The piston rod may be rotatable and axially moveable during dose delivery. However, movement of the drive member in the direction opposite to the delivery direction may not be converted into movement of the piston rod. Hence, the piston rod may be prevented from movement during a dose setting operation. The amount of friction exterted onto the drive member may regulate a speed of movement of the piston rod when the piston rod is moved in the delivery direction. Hence, by controlling the speed of movement of the drive member during dose delivery, the speed of movement of the piston rod, i.e. the injection speed, can be controlled as well. In this way, provision of a user friendly drug delivery device is facilitated.

According to one embodiment, the first and the second interaction member each comprise a surface. The surface may be inclined with respect to a longitudinal axis of the assembly. The inclined surfaces may face each other. The interaction members may be shaped wedge like. In the first position, the inclined surfaces may be arranged at a distance to one another. In the second position, the inclined surfaces may abut.

According to one embodiment, the respective inclined surface encloses an angle with the longitudinal axis. The angle may be between 5 degree and 45 degreee. The angle may amount to 10. 15 or 20 degree, for example. The amount of friction may depend on the enclosed respective angle. The greater the angle which the inclined surface encloses with the longitudinal axis, the greater the radial force exerted by the first interaction member onto the second interaction member when the second interaction member is moved towards the second position. The greater the angle, the greater the friction between the second interaction member and the drive member when the second interaction member is moved towards the second position. Thus, by means of the structure of the interaction members, the force onto the dive member and, accordingly, the speed of the drive member during dose delivery can be effectively controlled. By controlling the speed of the drive member during dose delivery, the speed of the piston rod can be controlled.

According to one embodiment, the interaction system further comprises an adjusting member. The adjusting member may be adapted and arranged to adjust a radial position of the first interaction member with respect to the drive member. The adjusting member may be adapted and arranged to adjust a radial position of the first interaction member with respect to the second interaction member. The amount of friction between the second interaction member and the drive member may depend on the radial position of the first interaction member. The further the first interaction member may be moved radially inwardly, the greater the friction onto the drive member. Hence, by adjusting the radial position of the interaction member, the speed of movement of the drive member during dose delivery is controllable.

The adjusting member may be shaped screw-like. The adjusting member may have a head that is configured to provide friction. The adjusting member may be in abutment, preferably permanent abutment, with the first interaction member. The adjusting member may be adapted and arranged to be operated by a user. The adjusting member allows adjusting the radial force exerted by the first interaction member onto the second interaction member and, thus, the friction generated by the interaction system. In this way, the speed of movement of the drive member is controlled in an effective and easy way.

According to one embodiment, the assembly further comprises a housing. The interaction system and, in particular the first and the second interaction member, are completely arranged within the housing. The housing may comprise an opening.. The adjusting member may be adapted and arranged to extend through the opening of the housing. In this way, the adjusting member can be directly accessed by the user for being operated. Thus, provision of a drug delivery device which can be easily operated is facilitated.

A further aspect relates to a drug delivery device. The device may comprise the previously mentioned assembly. In particular, the assembly may be implemented in the device. The device may further comprise a cartridge for holding a plurality of doses of the drug. The device or the assembly may further comprise an activation member adapted and arranged to be operated by a user for triggering a dose delivery operation of the assembly. The activation member is axially displaceable in the delivery direction for delivering a dose of the drug. The axial direction may be the direction along a main longitudinal axis of the assembly and, thus, of the device. The activation member is prevented from being rotated or tilted with respect to the housing for delivering a dose of the drug. The activation member is prevented from being moved in a radial direction with respect to a main longitudinal axis of the assembly and, thus, of the device for delivering a dose of the drug.

The device may further comprise the previously mentioned energy storing member. The energy storing member is adapted and arranged to store energy during a dose setting operation. The energy storing member is adapted and arranged to move the drive member and, thus, the piston rod in the delivery direction when the activation member is operated by the user. In particular, the energy storing member may be adapted and arranged to cause movement of the drive member during a delivery operation, wherein movement of the drive member is converted into movement of the piston rod. When the activation member is operated, the drive member and, thus, the piston rod may be driven due to the energy stored in the energy storing member for delivering the dose. In particular, the activation member is configured to release the energy stored in the energy storing member. The device may be an automatic device. By means of the assembly, the user can control the speed of movement of the drive member and, thus, of the piston rod. In particular, he can adjust the speed to his individual needs. Accordingly, a very user friendly device is provided.

Of course, features described above in connection with different aspects and embodiments may be combined with each other and with features described below.

Further features and refinements become apparent from the following description of the exemplary embodiments in connection with the accompanying figures.
Figure 1 schematically shows a three-dimensional view of a drug delivery device,
Figure 2 schematically shows a sectional side view of a part of the drug delivery device of Figure 1,
Figure 3 schematically shows a sectional side view of a part of the drug delivery device of Figure 1,
Figures 4A and 4B schematically show a sectional view of a part of the drug delivery device of Figure 1.

Like elements, elements of the same kind and identically acting elements may be provided with the same reference numerals in the figures.

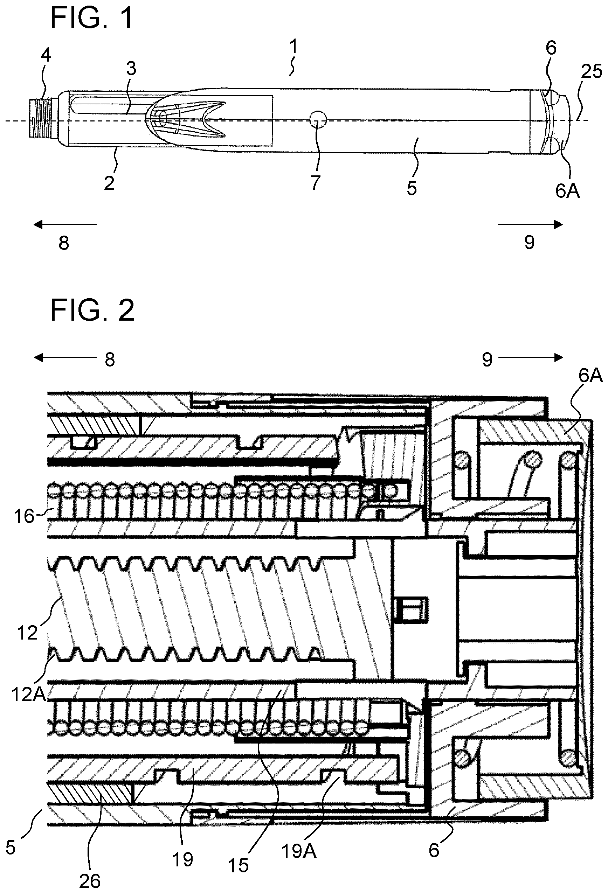

In Figure 1 a drug delivery device 1 is shown. The drug delivery device 1 comprises a housing 5. The housing 5 is adapted and arranged for protecting components of the device 1 arranged within the housing 5 from environmental influences. The drug delivery device 1 and the housing 5 have a distal end 8 and a proximal end 9. The term "distal end" designates that end of the drug delivery device 1 or a component thereof which is or is to be arranged closest to a dispensing end of the drug delivery device 1. The term "proximal end" designates that end of the device 1 or a component thereof which is or is to be arranged furthest away from the dispensing end of the device 1. The distal end 8 and the proximal end 9 are spaced apart from one another in the direction of an axis 25. The axis 25 may be the longitudinal axis or rotational axis of the device 1.

The drug delivery device 1 comprises a cartridge holder 2. The cartridge holder 2 comprises a cartridge 3. The cartridge 3 contains a drug 10, preferably a plurality of doses of the drug 10. The cartridge 3 is retained within the cartridge holder 2. The cartridge holder 2 stabilizes the position of the cartridge 3 mechanically. The cartridge holder 2 is connectable, e.g. by a threaded engagement or by a bayonet coupling, to the housing 5. The cartridge holder 2 and the housing 5 may be releasably or non-releasably connected to one another. In an alternative embodiment, the cartridge 3 may be directly connected to the housing 5. In this case, the cartridge holder 2 may be redundant.

The drug delivery device 1 may be a pen-type device, in particular a pen-type injector. The device 1 may be a re-usable device, which means that the cartridge 3 can be replaced, in particular during a reset operation, by a replacement cartridge for dispensing a plurality of doses from the replacement cartridge. Alternatively, the device 1 may be a disposable device. The device 1 can be configured to dispense variable doses of the drug 10. Alternatively, the device 1 may be a fixed dose device. The device 1 may be an auto-injection device. This means that a dose delivery operation of the device 1 may be initiated by the user by pressing an actuation member 6A, wherein upon pressing the actuation member 6A an energy stored in the device 1 is released for dispensing a dose of the drug 10. The auto-injector may be configured to substantially expel the entire content from the cartridge 3. Alternatively, the auto-injector may comprise a dose setting member 6 configured to determine the amount of drug 10 to be expelled.

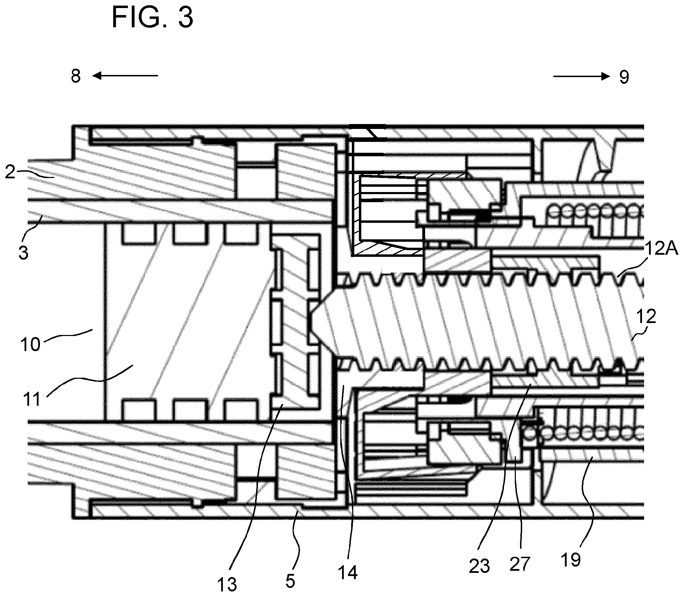

A bung 11 (see Figure 3) is slideably retained within the cartridge 3. The bung 11 seals the cartridge 3 proximally. Movement of the bung 11 in the distal direction with respect to the cartridge 3 causes the drug 10 to be dispensed from the cartridge 3. A needle assembly (not explicitly shown in the Figures) can be arranged at the distal end section of the cartridge holder 2, e.g. by means of an engagement means 4, e.g. a thread.

Figures 2 and 3 show a sectional side view of a part of the drug delivery device of Figure 1.

The device 1 comprises a piston rod 12. The piston rod 12 is configured to be moved in a distal or delivery direction in order to dispense a dose of the drug 10. The piston rod 12 is moved along the longitudinal axis 25 of the device 1. The longitudinal axis 25 is, thus, the axis of movement of the piston rod 12. The piston rod 12 is configured to move the bung 11 arranged in the cartridge 3 towards the dispensing end of the drug delivery device 1. The piston rod 12 comprises a bearing member 13 which is in contact with the bung 11. The piston rod 12 is configured as a lead screw. The piston rod 12 comprises an outer thread 12A. The thread 12A is arranged along an outer surface of the piston rod 12.

The device 1 may further comprise a drive feature 23. The drive feature 23 may comprise a spline nut. The drive feature 23 is engaged with the piston rod 12. In particular, the drive feature 23 comprises splines, which are engaged with axial grooves of the piston rod 12. Thus, the drive feature 23 is rotationally fixed but axially moveable with respect to the piston rod 12. The drive feature 23 and, thus, the piston rod 12 may be rotatable during a dose delivery operation. In an alternative embodiment, the drive feature 23 and the piston rod 12 may be prevented from rotational movement with respect to the housing 5.

The device 1 further comprises a guide member 14. The piston rod 12 is guided through the guide member 14. The guide member 14 is arranged around the piston rod 12. The guide member 14 may be a nut member. The guide member 14 is preferably configured as a thread nut. The guide member 14 is in threaded engagement, preferably in permanent threaded engagement, with the piston rod 12. For this purpose, the guide member 14 comprises an inner thread which is in engagement with the outer thread 12A of the piston rod 12. The guide member 14 is secured against movement with respect to the housing 5.

When the drive feature 23 rotates during a dose delivery operation, the piston rod 12 is also rotated. Due to the threaded engagement between the piston rod 12 and the guide member 14, rotation of the piston rod 12 causes the piston rod 12 to move in the distal direction. The distal direction may be the delivery direction. Thereby, the bung 11 is moved in the distal direction for dispensing a dose of the drug 10. In an alternative embodiment (not explicitly shown), the piston rod 12 may be prevented from rotation with respect to the housing 5. In this embodiment, the delivery movement of the piston rod 12 may be an axial movement in the distal direction. In this case, an outer thread of the piston rod 12 may be redundant.

Prior to dose delivery, the required dose of drug 10 is set by means of a dose setting mechanism. Common designs of dose setting mechanisms comprise a number of tubular or sleeve-like members such as a dose setting sleeve, a dose indicating sleeve, a drive sleeve and/or a ratchet sleeve. Such sleeves are often accommodated within and connected to each other.

The device 1 and, in particular the dose setting mechanism, comprises a dose setting member 6. The dose setting member 6 may be shaped sleeve-like. The dose setting member 6 is configured for setting a dose of the drug 10. The dose setting member 6 can be rotated by a user for setting a dose. Rotation of the dose setting member 6 during dose delivery may, however, be prevented. The device 1 further comprises a drive shaft 15. By rotating the dose setting member 6 for setting a dose, the drive shaft 15 is also rotated. In particular, the drive shaft 15 may be rotationally fixed to the dose setting member 6 during the dose setting operation due to a splined connection.

The device 1 further comprises a rotation member 19. The rotation member 19 may be a drve member. The rotation member 19 is rotatable and axially moveable with respect to the housing 5 during setting and delivering a dose of the drug 10. The rotation member 19 is shaped sleeve-like. The rotation member 19 is arranged concentrically around the drive shaft 15. The rotation member 19 may be fixed to the drive shaft 15, e.g. by a snap-fit connection. The rotation member 19 is axially fixed with respect to the drive shaft 15. Rotation of the drive shaft 15 during a dose setting operation causes rotation of the rotation member 19. For this purpose the rotation member comprises a thread 19A which may engage an inner thread of the housing 5 (not explicitly shown). The direction in which the drive shaft 15 and the rotation member 19 are rotated during dose setting, i.e. a first rotational direction, may be a clockwise direction. When the rotation member 19 is rotated during dose setting, the rotation member 19 moves in the proximal direction with respect to the housing 5.

The device further comprises a number sleeve 26. The number sleeve 26 may be a further drive member. The number sleeve 26 is rotatable and axially moveable with respect to the housing 5 during setting and delivering a dose of the drug 10. The number sleeve 26 is configured to indicate the amount of a set dose to a user. For example, the amount of a set dose may be shown through a window (not explicitly shown) in the housing 5. The number sleeve 26 is rotationally fixed, but axially moveable with respect to the rotation member 19. For example, the number sleeve 26 may comprise splines at its inner circumference which may engage in corresponding grooves of the rotation member 19. The number sleeve 26 is arranged concentrically around the rotation member 19. Furthermore, the number sleeve 26 is in threaded engagement with the housing 5. During the setting of a dose, the number sleeve 26 is rotated by the rotation member 19 in the first rotational direction. Thereby, the number sleeve 26 is forced to move in the proximal direction because of its threaded engagement with the housing 7.

To improve the user comfort the device 1 comprises a power assistance, in particular an energy storing member 16. The energy storing member 16 may be a coil spring. The energy storing member 16 may be a torsion spring. When the dose setting member 6 and, thus, the rotation member 19 is rotated during dose setting, the energy storing member 16 is compressed, such that energy is stored in the energy storing member 16. Thereby, a clicking noise is produced e.g. by a single ratchet on a ratchet sleeve (not explicitly shown in the Figures).

The device 1 further comprises a locking member 27. The locking member 27 is rotationally fixed with respect to the housing 5 during the setting of a dose, e.g. by means of a splined connection. The rotation member 19 is engaged with the locking member 27, e.g. by a toothed connection such that the rotation of the rotation member 19 in the first rotational direction is allowed during the dose setting operation.

The device 1 further comprises the previously mentioned actuation member 6A. In order to dispense a dose, the actuation member 6A is operated by a user. The actuation member 6A may comprise a button. Once pressing the actuation member 6A, the energy storing member 16 is released and drives the piston rod 12 to deliver a dose of the drug 10 from the cartridge 3. The user of the device 1 does, thus, not have to provide the force for dispense.

In particular, when the actuation member 6A is actuated, e.g. moved in a distal direction, the drive shaft 15 is also moved in a distal direction. Thereby, the drive shaft 15 is moved out of engagement with the dose setting member 6. When the drive shaft 15 is moved in the distal direction, the rotation member 19 and the locking member 27 are also moved in a distal direction. Thereby, the locking member 27 is disengaged from its engagement with the housing 5. In particular, the locking member 27 is allowed to rotate with respect to the housing 5 when the actuation member 6A is actuated by a user. When the locking member 27 is allowed to rotate, the rotation member 19 and the number sleeve 26 are allowed to rotate, as well. When the locking member 27 is enabled to rotate with respect to the housing 5, the energy which is stored in the energy storing member 16 is released causing a rotation and an axial movement of the rotation member 19 and the number sleeve 26 in the distal direction. Rotation of the rotation member 19 and, thus, of the number sleeve 26 causes rotation of the drive feature 23 and, thus, rotational movement of the piston rod 12 in the distal direction for dispensing a dose of the drug 10.

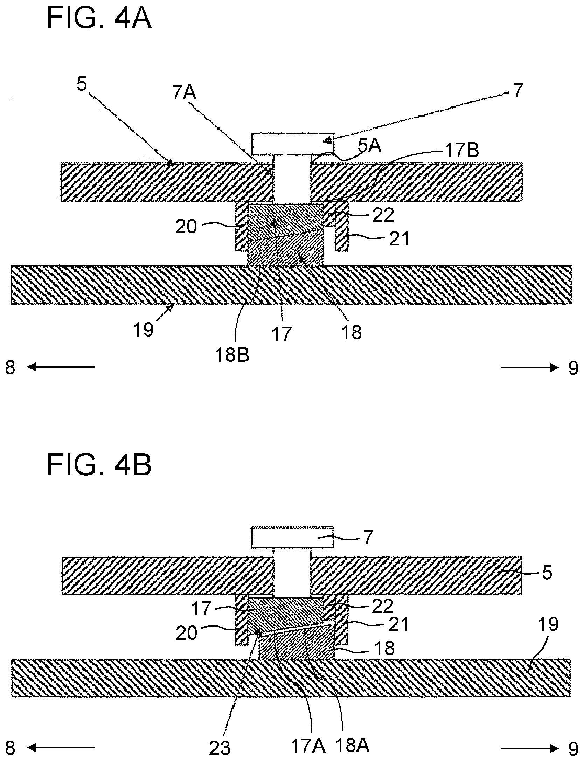

Figures 4A and 4B show a sectional view of a part of the drug delivery device of Figure 1.

In conventional drug delivery devices, the user has no control over the speed with which the piston rod 12 is moved during the dose delivery operation, i.e. the injection speed. In particular, when the above mentioned locking member 27 is released, the injection speed is independent from the pressed actuation member 6A. However, it is desirable for the user to control the injection speed, e.g. to reduce the pain when the drug 10 is injected into the tissue.

For this purpose, the device 1 comprises an interaction system. The interaction system may be a wedge system. The interaction system comprises a first interaction member 17. The first interaction member 17 is shaped wedge like. The first interaction member 17 comprises an inclined surface 17A. The inlined surface 17A is inclined with respect to the longitudinal axis 25. In particular, the inclined surface 17A encloses an angle with the longitudinal axis 25. The angle may be smaller than 45 degree. The angle may amount to 5, 10, 15 or 20 degree, for example.

The interaction system comprises a second interaction member 18. The second interaction member 18 is shaped wedge like. The second interaction member 18 comprises an inclined surface 18A. The inlined surface 18A is inclined with respect to the longitudinal axis 25. In particular, the inclined surface 18A encloses an angle with the longitudinal axis 25. The angle may be smaller than 45 degree. The angle may amount to 5, 10, 15 or 20 degree, for example.

The inclined surface 17A of the first interaction member 17 and the inclined surface 18A of the second interaction member 18 face each other. The angles which the inclined surfaces 17A, 18A each enclose with the longitudinal axis 25 may be equal.

The respective interaction member 17, 18 comprises a surface 17B, 18B which is opposite to the respective inclined surface 17A, 18A. Said surface 17B, 18B is arranged parallel to the longitudinal axis 25. The interaction members 17, 18 are arranged within the housing 5. In particular, they are arranged completely within the housing 5. The interaction members 17, 18 are arranged between an inner surface of the housing 5 and an outer surface of the rotation member 19. Alternatively (not explicitly shown), the interaction members 17, 18 are arranged between the inner surface of the housing 5 and an outer surface of the number sleeve 26. The second interaction member 18 is in direct mechanical contact with the outer surface of the rotation member 19 or of the number sleeve 26. The second interaction member 18 abuts, preferably permanently abuts, the outer surface of the rotation member 19 or of the number sleeve 26. The second interaction member 18 may be slidably arranged on the outer surface of the rotation member 19 or of the number sleeve 26.

The interaction members 17, 18 are moveable with respect to one another. The interaction members 17, 18 are moveable with respect to the rotation member 19 and to the number sleeve 26. In particular, the interaction members 17, 18 are moveable with respect to the housing 5. The first interaction member 17 is moveable in the radial direction with respect to the housing 5 and with respect to the second interaction member 18. The radial direction is that direction which is perpendicular to the delivery direction of the piston rod 12. The radial direction is, in particular, perpendicular to the longitudinal axis 25.

For adjusting a radial position of the first interaction member 17, the interaction system comprises an adjusting member 7. The adjusting member 7 is moveable in a radial direction with respect to the housing 5. The adjusting member 7 is rotatable with respect to the housing 5. The adjusting member 7 is rotatable around an axis which is perpendicular to the longitudinal axis 25. The adjusting member 7 may comprise a head portion and a body portion. The adjusting member 7 may be a screw or a screw-like member. The adjusting member 7 and, in particular, the body portion may comprise a thread. The adjusting member 7 can be operated by the user. For this purpose, the housing 5 comprises an opening 5A. The adjusting member 7 protrudes in the radial outward direction through the opening 5A of the housing 5. In particular, at least the head portion of the adjusting member 7 protrudes from the housing 5. Thus, the adjusting member 7 can be directly accessed by the user form an outside of the housing 5. The opening 5A may comprise a thread for enabling a threaded engagement with the adjusting member 7. In this way, the adjusting member 7 can be rotated in either direction, i.e. clockwise and anticlockwise.

The adjusting member 7, in particular the body portion abuts, preferably permanently abuts, with the non-inclined surface 17B of the first interaction member 17. Thus, when the adjusting member 7 is rotated in a first direction, e.g. radially inwardly, the adjusting member 7 will act onto the first interaction member 17 for moving the first interaction member 17 radially inwardly. The adjusting member 7 exerts a radial force onto the first interaction member 17. When the adjusting member 7 is rotated in a second direction, e.g. radially outwardly, the radial force onto the first interaction member 17 is at least partly released and the first interaction member 17 can move radially outwardly.

The first interaction member 17 is prevented from axial movement with respect to the housing 5. For this purpose, the interaction system comprises a first stop member 20 and a third stop member 22. The respective stop member 20, 22 may comprise a protrusion or a flange. The respective stop member 20, 22 protrudes from the housing 5, in particular from the inner surface of the housing 5, in the radial inward direction. The first stop member 20 protrudes further in the radial inward direction than the third stop member 22. In particular, the first stop member 20 comprises a greater length as compared to the third stop member 22. The first interaction member 17 comprises two side-faces. The respective side-face abuts, preferably permanently abuts, the first and third stop member 20, 22. Mechanical cooperation with the stop members 20, 22 prevents the first interaction member 17 from axial movement with respect to the housing 5. Moreover, mechanical cooperation of the first interaction member 17 with the first and third stop member 20, 22 prevents the first interaction member 17 from rotating with respect to the housing 5.

The second interaction member 18 is moveable with respect to the housing 5 in a radial direction. In particular, when the first interaction member 17 is moved by means of the adjusting member 7, the second interaction member 18 is moved due to mechanical cooperation with the first interaction member 17 in case the interaction members 17, 18 are positioned such that they abut which is explained later on in detail. In other words, when a radial force is exerted onto the first interaction member 17 by means of the adjusting member 7, the first interaction member 17 exerts a radial force onto the second interaction member 18 in case the interaction members 17, 18 are positioned such that they abut. Radial movement of the second interaction member 18 in the outward direction is limited by the first interaction member 17. Radial movement of the second interaction member 18 in the inward direction is limited by mechanical cooperation with the rotation member 19 or the number sleeve 26.

The second interaction member 18 is moveable with respect to the housing 5 and with respect to the first interaction member 17 in the distal and in the proximal direction. The second interaction member 18 is moveable between a first position, i.e. a proximal position (see Figure 4B), and a second position, i.e. a distal position (see Figure 4A). The second interaction member 18 may be moved towards the first position during a dose setting operation of the device 1. The second interaction member 18 may be moved towards the second position during a dose delivery operation of the device 1. The second interaction member 18 is axially moveable due to frictional forces between the second interaction member 18 and the rotation member 19 (or the number sleeve 26).

The interaction system further comprises a second stop member 21. The second stop member 21 is arranged adjacent to the third stop member 22 in the proximal direction. The second stop member 21 may comprise a protrusion or a flange. The second stop member 21 protrudes from the housing 5, in particular from the inner surface of the housing 5, in the radial inward direction. The second stop member 21 protrudes further in the radial inward direction than the third stop member 22. The second stop member 21 protrudes in the radial inward direction for the same amount as the first stop member 20. Accordingly, the first and second stop member 20, 21 have an equal length. The second interaction member 18 is positioned between the first and the second stop member 20, 21. However, the second interaction member 18 is not in permanent abutment with the first and second stop member 20, 21. Rather, the second interaction member 18 is axially moveable between the first and the second stop member 20, 21.

When the second interaction member 18 is arranged in the first position, the second interaction member 18 abuts with the second stop member 21. When the second interaction member 18 is arranged in the second position, the second interaction member 18 abuts with the first stop member 20. When the second interaction member 18 is moved between the first position and the second position, it may be free from abutment with the first and second stop member 20, 21.

When the second interaction member 18 is arranged in the first position (see Figure 4B), the interaction members 17, 18 are preferably free from mechanical cooperation. In particular, the inclined surfaces 17A, 18B may be free from abutment. Whether the interaction members 17, 18 mechanically cooperate when the second interaction member 18 is in the first position, depends on the radial position of the first interaction member 17. The more the first interaction member 27 is positioned radially inwardly (due to operating the adjusting member 7), the more problabe mechanical cooperation occurs when the second interaction member 18 is in the first position. However, it is preferred that, in the first position, there is no mechanical cooperation between the first and the second interaction member 17, 18, as shown in Figure 4B. Thus, in the first position, no radial force is exerted onto the second interaction member 18 and, thus, onto the rotation member 19 (or number sleeve 26).

When there is no force exerted onto the sceond interaction member 18, the rotation member 19 or the number sleeve 26 may be moveable with respect to the second interaction member 18 without the second interaction member 18 exerting a considerable force onto the respective member. Accordingly, when the second interaction member 18 is arranged in the first position, the rotation member 19 and the number sleeve 26 may be moveable in the proximal direction with respect to the second interaction member 18 for performing a dose setting operation without being impeded by the second interaction member 18.

When the second interaction member 18 is arranged in the second position, the interaction members 17, 18 preferably mechanically cooperate (see Figure 4A). In particular, the inclined surfaces 17A, 18B may be in abutment. In this position, the first interaction member 17 exerts a radially inwardly directed force onto the second interaction member 18. The amount of the force depends of the radial position of the adjusting member 7 and, thus, of the first interaction member 7. The more the first interaction member 7 is moved radially inwardly, the greater the force exerted onto the second interaction member 18.

The amount of force further depends on the axial position of the second interaction member 18. The closer the second interaction member 18 is arranged to the second position, the greater may be the force onto the second interaction member 18. The greater the force exerted onto the second interaction member 18, the greater the force exerted onto the rotation member 19 (or the number sleece 16) and, thus, the greater the friction between the second interaction member 18 and the rotation member 19 (or the number sleeve 26).

The amount of friction between the second interaction member 18 and the rotation member 19 (or the number sleeve 26) regulates a speed of movement of the rotation member 19 (or the number sleeve 26) during dose delivery. As movement during dose delivery is converted into movement of the piston rod 12, the amount of friction between the second interaction member 18 and the rotation member 19 (or the number sleeve 26) regulates a speed of the piston rod 12 when the piston rod 12 is moved in the delivery direction. Thus, the interaction system functions as an automatical break of the device 1, which is now explained in detail:
For setting a dose of the drug 10, the dose setting member 6 is rotated which results in rotational and proximal movement of the rotation member 19 and the number sleeve 26 as described above. Due to the proximal movement of the rotation member 19 and the number sleeve 26, the second interaction member 18 is forced to move in the proximal direction into the first position (Figure 4B) due to frictional forces. In this position, the first and second interaction member 17, 18 are free fom mechanical cooperation. In this position, the second interaction member 18 does not exert a force, in particular a radial force, onto the rotation member 19 (or, according to the alternative embodiment, onto the number sleeve 26). Accordingly, the interaction system does not influence, in particular hamper, the dose setting operation. Hence, the adjustable break is automatically released during dose setting.

For dose delivery, the activation member 6A is pushed distally which results in rotational and distal movement of the rotation member 19 and the number sleeve 26 as described above. Movement of the rotation member 19 (number sleeve 26) is converted into movement of the piston rod 12 in the delivery direction. Due to the distal movement of the rotation member 19 and the number sleeve 26, the second interaction member 18 is forced to move in the distal direction into the second position (Figure 4A) due to frictional forces. Movement of the second interaction member 18 is stopped by the first stop member 20 or, alternatively by the inclined surface 17A of the first interaction member 17 if the first interaction member 17 has been moved far enough in the radial inward direction such that the inclined surface 18A of the second interaction member 18 abuts the inclined surface 17A of the first interaction member 17 before the second interaction member 18 abuts the first stop member 20.

In this position, the first and second interaction member 17, 18 abut in any case and the first interaction member 17 pushes the second interaction member 18 radially inwardly. In this position, the second interaction member 18 exerts a force, in particular a radial force, onto the rotation member 19 (or, according to the alternative embodiment, onto the number sleeve 26). When the second interaction member 18 exerts the radial force onto the rotation member 19 (or, according to the alternative embodiment, onto the number sleeve 26), a speed of movement of the rotation member 19 (or, according to the alternative embodiment, onto the number sleeve 26) is reduced. In particular, the speed of movement is reduced as compared to the speed of movement during the dose setting operation, for example. The speed of movement is reduced as compared to the speed of movement when the second interaction member 18 is not in the second position. A reduced speed of movement of the rotation member 19 (or, according to the alternative embodiment, of the number sleeve 26) results in a reduced speed of movement of the piston rod 12 in the delivery direction as described above.

Accordingly, by adjusting the radial position of the first interaction member 17 by means of the adjusting member 7, the user can influence the friction onto the rotation member 19 / number sleeve 26 and, thus, the speed of movement of the piston rod 12 during dose delivery. The user may adjust the adjusting member 7 only once and the speed of movement of the piston rod 12 will stay the same during the whole lifetime of the device 1.

### Reference Numerals

- 1: Drug delivery device
- 2: Cartridge holder
- 3: Cartridge
- 4: Engagement means
- 5: Housing
- 6A: Activation member
- 6: Dose setting member
- 7: Adjusting member
- 7A: Thread
- 8: Distal end
- 9: Proximal end
- 10: Drug
- 11: Bung
- 12: Piston rod
- 12A: Thread
- 13: Bearing member
- 14: Guide member
- 15: Drive shaft
- 16: Energy storing member
- 17: First interaction member
- 17A: Inclined surface
- 17B: Surface
- 18: Second interaction member
- 18A: Inclined surface
- 18B: Surface
- 19: Rotation member
- 19A: Thread
- 20: First stop member
- 21: Second stop member
- 22: Third stop member
- 23: Drive feature
- 25: Axis
- 26: Number sleeve
- 27: Locking member

## Claims

1. An assembly for a drug delivery device (1) comprising
- at least one drive member (19, 26) adapted and arranged to be moved in a delivery direction during a dose delivery operation of the assembly and in a direction opposite to the delivery direction during a dose setting operation of the assembly,
- an energy storing member (16) adapted and arranged to store energy and to cause movement of the drive member (19, 26) in the delivery direction, and
- an interaction system adapted and arranged to generate a friction onto the drive member (19, 26), wherein an amount of friction regulates a speed of movement of the drive member (19, 26) when drive member (19, 26) is moved in the delivery direction,
- wherein the interaction system comprises a first interaction member (17) and a second interaction member (18),
- wherein the interaction members (17, 18) are moveable with respect to one another and with respect to the drive member (19, 26), and
- wherein the second interaction member (18) is configured to exert a radial force onto the drive member (19, 26) such that the speed of movement of the drive member (19, 26) is reduced when the drive member (19, 26) is moved in the delivery direction during a dose delivery operation,
**characterized in that**
- the second interaction member (18) is axially moveable with respect to the first interaction member (17) between a first position and a second position,
- the second interaction member (18) is adapted and arranged to be moved towards the first position during a dose setting operation of the assembly and to be moved towards the second position during a dose delivery operation of the assembly, and
the interaction system is adapted and arranged such that the amount of friction increases when the second interaction member (18) is moved towards the second position.

2. The assembly according to claim 1,
wherein the first interaction member (17) is adapted to be moved with respect to the second interaction member (18) and to the drive member (19, 26) in a direction which is perpendicular to the delivery direction and, wherein the amount of friction depends on a radial position of the first interaction member (17).

3. The assembly according to claim 1 or claim 2,
wherein the interaction system further comprises at least one stop member (20, 21), wherein the stop member (20, 21) is adapted and arranged to limit the axial movement of the second interaction member (18) between the first and the second position.

4. The assembly according to any of claims 1 to 3,
wherein, when the second interaction member (18) is arranged in the second position, the first and second interaction members (17, 18) are in mechanical contact with one another, and wherein, when the second interaction member (18) is arranged in the first position, the first and second interaction members (17, 18) are free from mechanical contact with one another.

5. The assembly according to any of the previous claims,
further comprising a piston rod (12) adapted and arranged to be displaceable in the delivery direction for dispensing a dose of a drug (10) from the device (1), wherein movement of the drive member (19, 26) in the delivery direction is converted into movement of the piston rod (12) in the delivery direction, and wherein the amount of friction exterted onto the drive member (19, 26) regulates a speed of movement of the piston rod (12) when the piston rod (12) is moved in the delivery direction.

6. The assembly according to any of claims 1 to 5,
wherein the first and the second interaction member (17, 18) each comprise a surface (17A, 18A) which is inclined with respect to a longitudinal axis (25) of the assembly, and wherein the inclined surfaces (17A, 17B) face each other.

7. The assembly according to claim 6,
wherein the respective inclined surface (17A, 18A) encloses an angle with the longitudinal axis (25), and wherein the amount of friction depends on the enclosed respective angle.

8. The assembly according to any of claims 1 to 7,
wherein the interaction system further comprises an adjusting member (7) which is adapted and arranged to adjust a radial position of the first interaction member (17) with respect to the drive member (19, 26), wherein the adjusting member (7) is adapted and arranged to be operated by a user.

9. The assembly according to claim 8,
further comprising a housing (5), wherein the housing (5) comprises an opening (5A) and, wherein the adjusting member (7) is adapted and arranged to extend through the opening (5A) of the housing (5).

10. A drug delivery device comprising the assembly according to any of the previous claims, wherein the drug delivery device (1) further comprises a cartridge (3) for holding a plurality of doses of the drug (10) and an activation member (6A) adapted and arranged to be operated by a user for triggering a dose delivery operation of the assembly, wherein the energy storing member (16) is adapted and arranged to cause a movement of the drive member (19, 26) in the delivery direction when the activation member (16) is operated by the user.

## Patentansprüche

1. Anordnung für eine Medikamenten-Verabreichungsvorrichtung (1), umfassend
- mindestens ein Antriebsglied (19, 26), das dazu ausgebildet und angeordnet ist, während eines Dosisverabreichungsvorgangs der Anordnung in einer Verabreichungsrichtung und während eines Dosiseinstellvorgangs der Anordnung in einer der Verabreichungsrichtung entgegengesetzten Richtung bewegt zu werden,
- ein Energiespeicherglied (16), das dazu ausgebildet und angeordnet ist, Energie zu speichern und eine Bewegung des Antriebsglieds (19, 26) in der Verabreichungsrichtung zu bewirken, und
- ein Zusammenwirkungssystem, das dazu ausgebildet und angeordnet ist, eine Reibung auf das Antriebsglied (19, 26) zu erzeugen, wobei ein Grad an Reibung eine Bewegungsgeschwindigkeit des Antriebsglieds (19, 26) reguliert, wenn das Antriebsglied (19, 26) in die Verabreichungsrichtung bewegt wird,
- wobei das Zusammenwirkungssystem ein erstes Zusammenwirkungsglied (17) und ein zweites Zusammenwirkungsglied (18) umfasst,
- wobei die Zusammenwirkungsglieder (17, 18) zueinander und bezüglich des Antriebsglieds (19, 26) beweglich sind und
- wobei das zweite Zusammenwirkungsglied (18) dazu ausgestaltet ist, eine radiale Kraft auf das Antriebsglied (19, 26) auszuüben, so dass die Bewegungsgeschwindigkeit des Antriebsglieds (19, 26) reduziert wird, wenn das Antriebsglied (19, 26) während eines Dosisverabreichungsvorgangs in die Verabreichungsrichtung bewegt wird,
**dadurch gekennzeichnet, dass**
- das zweite Zusammenwirkungsglied (18) bezüglich des ersten Zusammenwirkungsglieds (17) zwischen einer ersten Position und einer zweiten Position axial beweglich ist,
- das zweite Zusammenwirkungsglied (18) dazu ausgebildet und angeordnet ist, während eines Dosiseinstellvorgangs der Anordnung zur ersten Position hin bewegt zu werden und während eines Dosisverabreichungsvorgangs der Anordnung zur zweiten Position hin bewegt zu werden, und das Zusammenwirkungssystem so ausgebildet und angeordnet ist, dass der Grad an Reibung zunimmt, wenn das zweite Zusammenwirkungsglied (18) zur zweiten Position hin bewegt wird.

2. Anordnung nach Anspruch 1,
wobei das erste Zusammenwirkungsglied (17) dazu ausgebildet ist, in einer senkrecht zu der Verabreichungsrichtung verlaufenden Richtung bezüglich des zweiten Zusammenwirkungsglieds (18) und des Antriebsglieds (19, 26) bewegt zu werden und wobei der Grad an Reibung von einer radialen Position des ersten Zusammenwirkungsglieds (17) abhängig ist.

3. Anordnung nach Anspruch 1 oder Anspruch 2,
wobei das Zusammenwirkungssystem ferner mindestens ein Anschlagsglied (20, 21) umfasst, wobei das Anschlagsglied (20, 21) dazu ausgebildet und angeordnet ist, die axiale Bewegung des zweiten Zusammenwirkungsglieds (18) zwischen der ersten und der zweiten Position zu begrenzen.

4. Anordnung nach einem der Ansprüche 1 bis 3,
wobei das erste und das zweite Zusammenwirkungsglied (17, 18) in mechanischem Kontakt miteinander stehen, wenn das zweite Zusammenwirkungsglied (18) in der zweiten Position angeordnet ist, und wobei das erste und das zweite Zusammenwirkungsglied (17, 18) nicht in mechanischem Kontakt miteinander stehen, wenn das zweite Zusammenwirkungsglied (18) in der ersten Position angeordnet ist.

5. Anordnung nach einem der vorhergehenden Ansprüche, ferner umfassend eine Kolbenstange (12), die zur Abgabe einer Dosis eines Medikaments (10) aus der Vorrichtung (1) in der Verabreichungsrichtung verschiebbar ausgebildet und angeordnet ist, wobei die Bewegung des Antriebsglieds (19, 26) in der Verabreichungsrichtung in eine Bewegung der Kolbenstange (12) in der Verabreichungsrichtung umgewandelt wird und wobei der Grad der auf das Antriebsglied (19, 26) ausgeübten Reibung eine Bewegungsgeschwindigkeit der Kolbenstange (12) reguliert, wenn die Kolbenstange (12) in der Verabreichungsrichtung bewegt wird.

6. Anordnung nach einem der Ansprüche 1 bis 5,
wobei das erste und das zweite Zusammenwirkungsglied (17, 18) jeweils eine Fläche (17A, 18A) umfassen, die bezüglich einer Längsachse (25) der Anordnung geneigt ist, und wobei die geneigten Flächen (17A, 17B) einander zugewandt sind.

7. Anordnung nach Anspruch 6,
wobei die jeweilige geneigte Fläche (17A, 18A) einen Winkel mit der Längsachse (25) einschließt und wobei der Grad an Reibung von dem jeweiligen eingeschlossenen Winkel abhängt.

8. Anordnung nach einem der Ansprüche 1 bis 7,
wobei das Zusammenwirkungssystem ferner ein Verstellglied (7) umfasst, das dazu ausgebildet und angeordnet ist, eine radiale Position des ersten Zusammenwirkungsglieds (17) bezüglich des Antriebsglieds (19, 26) zu verstellen, wobei das Verstellglied (7) dazu ausgebildet und angeordnet ist, von einem Benutzer betätigt zu werden.

9. Anordnung nach Anspruch 8,
ferner umfassend ein Gehäuse (5), wobei das Gehäuse (5) eine Öffnung (5A) umfasst und wobei das Verstellglied (7) dazu ausgebildet und angeordnet ist, sich durch die Öffnung (5A) des Gehäuses (5) zu erstrecken.

10. Medikamenten-Verabreichungsvorrichtung, umfassend die Anordnung nach einem der vorhergehenden Ansprüche, wobei die Medikamenten-Verabreichungsvorrichtung (1) ferner eine Kartusche (3) zur Aufnahme einer Vielzahl von Dosen des Medikaments (10) und ein Aktivierungsglied (6A) umfasst, das dazu ausgebildet und angeordnet ist, zum Auslösen eines Dosisverabreichungsvorgangs der Anordnung von einem Benutzer betätigt zu werden, wobei das Energiespeicherglied (16) dazu ausgebildet und angeordnet ist, eine Bewegung des Antriebsglieds (19, 26) in der Verabreichungsrichtung zu veranlassen, wenn das Aktivierungsglied (16) vom Benutzer betätigt wird.

## Revendications

1. Ensemble pour un dispositif d'administration de médicament (1), comprenant
- au moins un organe d'entraînement (19, 26) conçu et disposé pour être déplacé dans une direction d'administration au cours d'une opération d'administration de dose de l'ensemble et dans une direction opposée à la direction d'administration au cours d'une opération de définition de dose de l'ensemble,
- un organe de stockage d'énergie (16) conçu et disposé pour stocker de l'énergie et pour provoquer un déplacement de l'organe d'entraînement (19, 26) dans la direction d'administration, et
- un système d'interaction conçu et disposé pour générer un frottement sur l'organe d'entraînement (19, 26), une quantité de frottement régulant une vitesse de déplacement de l'organe d'entraînement (19, 26) lorsque l'organe d'entraînement (19, 26) est déplacé dans la direction d'administration,
- le système d'interaction comprenant un premier organe d'interaction (17) et un second organe d'interaction (18),
- les organes d'interaction (17, 18) étant déplaçables l'un vis-à-vis de l'autre et par rapport à l'organe d'entraînement (19, 26), et
- le second organe d'interaction (18) étant configuré pour exercer une force radiale sur l'organe d'entraînement (19, 26) de telle sorte que la vitesse de déplacement de l'organe d'entraînement (19, 26) soit réduite lorsque l'organe d'entraînement (19, 26) est déplacé dans la direction d'administration au cours d'une opération d'administration de dose,
**caractérisé en ce que**
- le second organe d'interaction (18) est déplaçable axialement par rapport au premier organe d'interaction (17) entre une première position et une seconde position,
- le second organe d'interaction (18) est conçu et disposé pour être déplacé en direction de la première position au cours d'une opération de définition de dose de l'ensemble et pour être déplacé en direction de la seconde position au cours d'une opération d'administration de dose de l'ensemble, et
le système d'interaction est conçu et disposé de telle sorte que la quantité de frottement augmente lorsque le second organe d'interaction (18) est déplacé en direction de la seconde position.

2. Ensemble selon la revendication 1,
dans lequel le premier organe d'interaction (17) est conçu pour être déplacé par rapport au second organe d'interaction (18) et à l'organe d'entraînement (19, 26) dans une direction qui est perpendiculaire à la direction d'administration et dans lequel la quantité de frottement dépend d'une position radiale du premier organe d'interaction (17).

3. Ensemble selon la revendication 1 ou 2,
dans lequel le système d'interaction comprend en outre au moins un organe d'arrêt (20, 21), dans lequel l'organe d'arrêt (20, 21) est conçu et disposé pour limiter le déplacement axial du second organe d'interaction (18) entre les première et seconde positions.

4. Ensemble selon l'une quelconque des revendications 1 à 3,
dans lequel, lorsque le second organe d'interaction (18) est disposé dans la seconde position, les premier et second organes d'interaction (17, 18) sont en contact mécanique l'un avec l'autre, et
dans lequel, lorsque le second organe d'interaction (18) est disposé dans la première position, les premier et second organes d'interaction (17, 18) sont hors de contact mécanique l'un vis-à-vis de l'autre.

5. Ensemble selon l'une quelconque des revendications précédentes,
comprenant en outre une tige de piston (12) conçue et disposée pour être déplaçable dans la direction d'administration pour la distribution d'une dose d'un médicament (10) à partir du dispositif (1), dans lequel le déplacement de l'organe d'entraînement (19, 26) dans la direction d'administration est converti en déplacement de la tige de piston (12) dans la direction d'administration, et dans lequel la quantité de frottement exercée sur l'organe d'entraînement (19, 26) régule une vitesse de déplacement de la tige de piston (12) lorsque la tige de piston (12) est déplacée dans la direction d'administration.

6. Ensemble selon l'une quelconque des revendications 1 à 5,
dans lequel les premier et second organes d'interaction (17, 18) comprennent chacun une surface (17A, 18A) qui est inclinée par rapport à un axe longitudinal (25) de l'ensemble, et dans lequel les surfaces inclinées (17A, 17B) se font face.

7. Ensemble selon la revendication 6,
dans lequel la surface inclinée (17A, 18A) respective délimite un angle avec l'axe longitudinal (25), et dans lequel la quantité de frottement dépend de l'angle respectif délimité.

8. Ensemble selon l'une quelconque des revendications 1 à 7,
dans lequel le système d'interaction comprend en outre un organe d'ajustement (7) qui est conçu et disposé pour ajuster une position radiale du premier organe d'interaction (17) par rapport à l'organe d'entraînement (19, 26), dans lequel l'organe d'ajustement (7) est conçu et disposé pour être actionné par un utilisateur.

9. Ensemble selon la revendication 8,
comprenant en outre un logement (5), dans lequel le logement (5) comprend une ouverture (5A) et dans lequel l'organe d'ajustement (7) est conçu et disposé pour s'étendre à travers l'ouverture (5A) du logement (5).

10. Dispositif d'administration de médicament comprenant l'ensemble selon l'une quelconque des revendications précédentes, le dispositif d'administration de médicament (1) comprenant en outre une cartouche (3) destinée à renfermer une pluralité de doses du médicament (10) et un organe d'activation (6A) conçu et disposé pour être actionné par un utilisateur afin de déclencher une opération d'administration de dose de l'ensemble, dans lequel l'organe de stockage d'énergie (16) est conçu et disposé pour provoquer un déplacement de l'organe d'entraînement (19, 26) dans la direction d'administration lorsque l'organe d'activation (16) est actionné par l'utilisateur.
